Europäisches Patentamt

(19) European Patent Office

Office européen des brevets

(11) Publication number: **0 304 266**
**A2**

# (12) EUROPEAN PATENT APPLICATION

(21) Application number: 88307575.6

(22) Date of filing: 16.08.88

(51) Int. Cl.⁴: **G 01 N 25/36**
**G 01 N 33/22**

(30) Priority: 17.08.87 US 86906

(43) Date of publication of application:
**22.02.89 Bulletin 89/08**

(84) Designated Contracting States:
**AT BE CH DE ES FR GB GR IT LI LU NL SE**

(71) Applicant: **HART SCIENTIFIC, INCORPORATED**
**177 West 300 South**
**Provo Utah 84601 (US)**

(72) Inventor: **Hart, Roger M.**
**960 East 900 North**
**Mapleton Utah 84663 (US)**

**Lewis, Edwin A.**
**149 North 300 West**
**Springville Utah 84663 (US)**

**Schurig, Alma K.**
**870 East Walnut Avenue**
**Provo Utah 84604 (US)**

(74) Representative: **Geldard, David Guthrie et al**
**URQUHART-DYKES AND LORD Tower House Merrion Way**
**Leeds West Yorkshire LS2 8PA (GB)**

(54) **Catalytic gas calorimeter systems and methods.**

㉗ A novel catalytic gas calorimeter system and method. The system comprises a catalytic reactor which includes a catalyst capable of oxidizing the sample gas. The catalyst is configured within the reactor so as to maximize the catalyst surface area available to the sample gas, such as, for example, by depositing the catalyst on a bed of small beads of porous surface structure or on a porous monolithic substrate. The reactor temperature and space velocities are maintained at a level which will ensure substantially complete oxidation of the sample gas.

The catalytic reactor of the present invention is surrounded by one or more temperature shields to isolate the reactor from the surrounding environment. A sensor device is positioned within the reactor to measure the temperature within the reactor. The calorimeter system also includes means for measuring and controlling the volume of gas and air entering the catalytic reactor and means for measuring the relative humidity of the gas stream leaving the catalytic reactor. These measurements are then used to calculate the net heating value (NHV), gross heating value (GHV) and the specific gravity of the sample gas under consideration.

FIG.1

**EP 0 304 266 A2**

**POOR QUALITY**

Bundesdruckerei Berlin

## Description

## BACKGROUND

### 1. The Field of the Invention

This invention relates to calorimeter devices and methods. More particularly, this invention relates to a novel calorimeter system and method employing catalytic gas combustion for measuring the net heating value, the gross heating value, and the specific gravity of a sample gas.

### 2. The Background Art

It is often necessary or desirable to measure the heating value (enthalpy) and/or density (specific gravity) of a sample gas. For example, natural gas suppliers frequently mix two or more streams of gas together in providing natural gas to a customer. Importantly, equal volumes of different gas streams may have significantly different heating values (that is, two gas streams may differ significantly in their capacity to generate heat during combustion). Therefore, both natural gas suppliers and major natural gas consumers are typically interested in knowing the heating value of the gas being delivered, rather than merely the quantity (volume) of gas delivered. Gas suppliers and major gas consumers also commonly want to know the density (specific gravity) of the gas being delivered, which can provide useful information about the composition of the gas.

A device commonly used for measuring the heating value of a gas is a calorimeter. Generally speaking, a gas calorimeter is a device which comprises an environmentally controlled reaction chamber in which the gas can be oxidized (burned in the presence of oxygen). The heat generated as a result of oxidation of the gas is then measured to determine the heating value of the gas per unit volume.

Conventional calorimeters are typically operated in one of two ways. First, the calorimeter may be operated isothermally (that is, the flow of air and gas into the reaction chamber is controlled so as to control the rate of oxidation of the gas and thereby maintain a substantially constant temperature within the reaction chamber). Second, the calorimeter may be operated in an isoperibol fashion (that is, the rate of flow of gas and air into the reaction chamber is maintained substantially constant while detecting changes in temperature within the reaction chamber).

For isothermal operation, conventional calorimeters may, for example, introduce a sample gas into the reaction chamber at a controlled volumetric flow rate. Air is then separately introduced into the reaction chamber, and oxidation of the sample gas in the presence of the air is induced by a flame. The flow rate of the gas into the reaction chamber is carefully controlled so as to control the rate of oxidation of the sample gas and thereby maintain a substantially constant temperature within the reaction chamber. Since the flow rate of the gas which is required to maintain such a constant temperature will depend upon the heating value of the sample gas, the flow rate of the gas can be periodically measured in order to calculate the heating value of the sample gas.

When configured so as to operate in an isoperibol fashion, conventional calorimeters may oxidize both a known volume of a standard gas having a known heating value and an equal volume of a sample gas. The temperature within the reaction chamber resulting from oxidation of the sample gas is then compared with the temperature resulting from oxidation of the standard gas. The heating value of the sample gas may then be approximated using conventional interpolation methods.

Conventional calorimeters such as those described above have been used for many years and have been developed to the point that they can give quite accurate results under usual operating conditions. Nevertheless, these conventional calorimeters suffer from a number of significant disadvantages which have not heretofore been overcome.

Perhaps the most readily noticeable disadvantage of prior art calorimeters is that they are generally quite large and expensive. The prior art calorimeters frequently cost in excess of Twenty-Five Thousand Dollars ($25,000) and may cost as much as Forty Thousand Dollars ($40,000). Prior art calorimeters often include up to 100 gallons of water which is used to thermally stabilise the component parts of the calorimeter device. The cost, size, and sensitivity to environmental conditions of prior art calorimeters has severely limited their usefulness at remote locations and has rendered them impractical for many potential users.

As noted above, the prior art calorimeters typically use a flame to oxidize the sample gas. This, of course, requires that the gas/air mixture in prior art calorimeters be flammable (that is, the gas/air mixture must be above the so-called lower inflammability limit for the sample gas). Moreover, flame oxidation takes place at relatively high temperatures (on the order of $2000°C$). Significantly, potential calorimeter installation sites include those which are commonly referred to as Class 1, Division II sites (sites at which an explosive gas may occasionally be present). It will be readily appreciated, therefore, that the use of a high temperature flame in prior art calorimeters creates a significant risk of explosion at such sites.

The use of a flame within prior art calorimeters also requires complex mechanical systems and significant gas flow to maintain the flame. The prior art calorimeters are also quite difficult to calibrate and maintain and must generally be installed in rooms having a constant air-conditioned temperature. These requirements generally necessitate hiring skilled operators to be responsible for prior art calorimeters.

When measuring the heating value of a gas, there are generally two separate measurements which are of interest: the net heating value (NHV) and the gross heating value (GHV). For hydrocarbon gases, the NHV is a measurement of the heat liberated in oxidizing the gas to produce the by-products of carbon dioxide gas and water vapor. The GHV, on the other hand, is equal to the NHV plus the heat released by the water vapor as it condenses into liquid form. For some applications, it is desirable to measure the NHV, while other applications may require an accurate measurement of the GHV. Unfortunately, prior art calorimeters typically measure either the NHV or the GHV, not both.

An alternate method of heating value measurement which is finding increasingly wide usage is gas chromatography. Using this method, the various chemical components of the sample gas are separated by selective absorption by passage through a column. The concentration of each separate component is then measured, and the heating value of the sample gas can then be calculated from the measured concentrations of the various chemical components.

While gas chromatography is generally considered much safer than conventional calorimeters, the equipment required for gas chromatography is still quite expensive. Gas chromatography devices also require frequent manual calibrations and a clean operating environment. Further, gas chromatography requires the analysis of discrete samples and does not lend itself to substantially continuous measurements as does conventional calorimetry. Therefore, those who need substantially continous heating value measurements still rely chiefly upon calorimeter devices.

## BRIEF SUMMARY AND OBJECTS OF THE INVENTION

In view of the foregoing, the present invention seeks to provide a gas calorimeter system and method which is capable of making sensitive heating value measurements but which can be small, inexpensive, and suitable for field installation where environmental control is not available.

According to a first aspect of the invention, a gas calorimeter comprises:
a reactor cell for oxidizing the sample gas;
a porous substrate positioned within the reactor cell;
a catalyst coating the porous substrate for oxidizing the sample gas;
means for metering a desired volume of the sample gas into the reactor cell;
means for metering a desired volume of air into the reactor cell; and
means for sensing the temperature within the reactor cell.

According to a second aspect of the invention a method for measuring the net heating value and the gross heating value of a sample of gas comprises the steps of:
metering a known volume of the sample gas, into a reactor cell;
oxidizing the sample gas within the reactor cell in the presence of an excess of air;
monitoring the temperature within the reactor cell; and
measuring the amount of water produced by oxidation of the sample gas.

The catalyst is desirably configured within the reactor so as to maximize the catalyst surface area available to the sample gas, such as, for example, by depositing the catalyst on a bed of small beads of porous surface structure or on a porous monolithic substrate. The reactor temperature and space velocities are preferably maintained at a level which will ensure substantially complete oxidation of the sample gas.

The catalytic reactor of the present invention is desirably surrounded by one or more temperature shields to isolate the reactor from the surrounding environment. A sensor device may be positioned within the reactor to measure the temperature within the reactor. The calorimeter system may also include means for measuring and controlling the volume or mass of gas and air entering the catalytic reactor and means for measuring the relative humidity of the exhaust gas stream leaving the catalytic reactor. These measurements can then be used to calculate the net heating value (NHV), the gross heating value (GHV) and the specific gravity of the sample gas under consideration.

These and other features of the present invention will become more fully apparent from the following description and appended claims, taken in conjunction with the accompanying drawings.

## BRIEF DESCRIPTION OF THE DRAWINGS

Figure 1 is a schematic illustration of one presently preferred embodiment of the gas calorimeter system of the present invention.

Figure 2 is a vertical cross-sectional view of a first presently preferred embodiment of the catalytic reactor of the present invention.

Figure 3 is a vertical cross-sectional view of a second presently preferred embodiment of the catalytic reactor of the present invention.

Figure 4 is a side elevation view of a third presently preferred embodiment of the catalytic reactor of the present invention, portions of the thermal shield being broken away to reveal the internal construction.

Figure 5 is a horizontal cross-sectional view of the embodiment of Figure 4 taken along lines 5-5 of Figure 4.

Figure 6 is a horizontal cross-sectional view of the top manifold of the catalytic reactor of Figure 4 taken along lines 6-6 of Figure 4.

Figure 7 is a horizontal cross-sectional view of the bottom manifold of the catalytic reactor of Figure 4 taken along lines 7-7 of Figure 4.

Figure 8 is a graphical illustration showing how various calculations can be accurately made in accordance with the present invention.

## DETAILED DESCRIPTION OF THE PREFERRED EMBODIMENTS

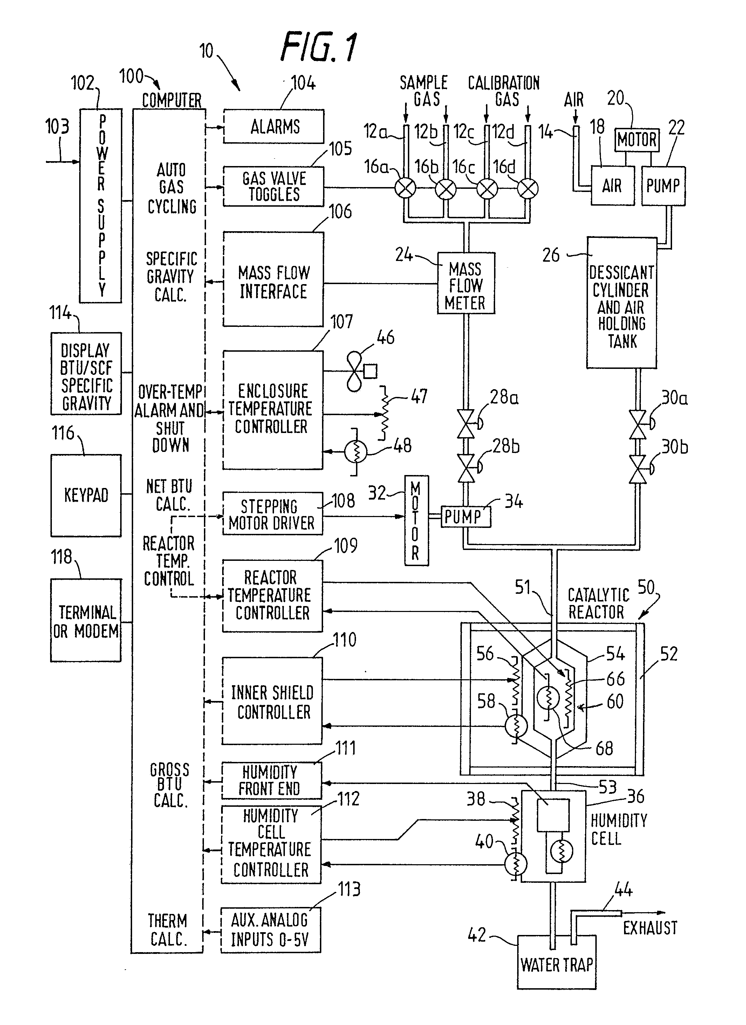

It will be readily appreciated that the components of the present invention, as generally described and illustrated in the figures herein, could be arranged and designed in a wide variety of different configurations Thus, the following more detailed description of the embodiments of the system and method of the present invention, as represented in Figures 1-8, is not intended to limit the scope of the invention, as claimed, but it is merely representative of one presently preferred embodiment of the invention.

The presently preferred embodiments of the invention will be best understood by reference to the accompanying drawings, wherein like parts are designated with like numerals throughout.

### 1. The Calorimeter System

One presently preferred embodiment of the calorimeter system of the present invention, designated generally at 10, is illustrated in its entirety in Figure 1. In gas calorimeter 10, both gas and air (in excess) are metered into a catalytic reactor 50, and the net energy produced in the combustion process is measured. From this energy measurement, the net heating value (NHV) of the gas can be determined. The gaseous combustion products, including water vapor, then exit through the reactor exhaust 53. Measurement of the amount of water produced (through measurement of the relative humidity of the exhaust) then allows accurate conversion of NHV to the gross heating value (GHV) of the gas.

Gas enters calorimeter system 10 through one or more inlets 12, and each inlet 12 can be connected to an appropriate gas supply by means which are well-known in the art. Advantageously, calorimeter system 10 may be calibrated using one or more calibration gases having known heating values and specific gravity. Accordingly, calorimeter system 10 may advantageously be provided with four inlets 12 as depicted in Figure 1. Inlets 12a and 12b may be connected to appropriate sample gas supplies. Inlets 12c and 12d, on the other hand, may each be connected to a suitable calibration gas supply.

Each inlet tube 12 is provided with a separate gas valve 16 which can be selectively opened and closed so as to regulate gas flow therethrough. Preferably, valves 16 are designed so as to be capable of electronic actuation, as will be discussed further below. After passing through valves 16, inlets 12 are connected to a mass flow meter 24. Mass flow meter 24 measures the rate at which combustion mass is flowing into calorimeter system 10 in some suitable units, such as, for example, grams per second. As discussed further below, the data obtained from mass flow meter 24 can be used to calculate the specific gravity of a gas without the use of a separate specific gravity meter.

The gas then flows through one or more regulators 28. Such regulators 28 may comprise a number of different devices. For example, regulators 28 may comprise suitable pressure regulators. In such case, an absolute pressure regulator could advantageously be used to minimize the effects of ambient pressure changes on gas flow rates. Alternatively, regulators 28 may comprise suitable flow metering devices, such as, for example, capillary tubes or adjustable flow metering valves.

From regulators 28, the gas flows to a volumetric gas metering means, such as, for example, a volumetric pump 34. Pump 34 is controlled by a stepping motor 32, thereby permitting the volume of gas entering catalytic reactor 50 to be very accurately measured and monitored. A suitable stepping motor 32 may, for example, be one of several commercially available models which provides approximately 7.5° per step and approximately 20 ounce-inches (250 Newton-Meters) of torque Alternatively, a suitable gas metering means could comprise a gas metering manifold with an appropriately sized sampling loop, and the flow of gas could be measured and regulated by controlling the number of "samples" provided per minute.

Air enters calorimeter system 10 through an inlet 14 into a suitable air space 18. The air is then pumped by pump 22 and motor 20 (which may, for example, comprise a 24 volt DC brushless diaphragm pump), into a suitable dessicant cylinder and air holding tank 26 where the air is dried and temporarily stored. The air then passes through one or more regulators 30 into catalytic reactor 50. As with regulators 28, regulators 30 may comprise suitable pressure regulators (absolute or gauge) or flow metering devices. Regulators 30 could also comprise suitable mass flow controllers for metering air on the basis of mass.

The gas supplies which are connected to inlets 12 will typically have a pressure of 12 to 20 pounds per square inch (83 to 138 KPa) gauge. The air entering calorimeter system 10 through inlet 14 is pressurized within holding tank 26 by pump 22 to a similar pressure level. The gas and air are then mixed so as to provide a gas to air volume ratio within the range of from approximately 1% to approximately 5%. This ratio ensures that there will be an excess of air in catalytic reactor 50, thereby enabling substantially all of the gas to be oxidized. Further, the above-noted gas to air volume ratio is below the lower inflammability limit for methane and most other potential sample gasses.

Advantageously, calorimeter system 10 is capable of operating with virtually any gas flow rate, including flow rates which are significantly lower than those typically required in prior art calorimeters. For example, pressure and flow regulators 30 may be adjusted so as to provide an air flow rate within the range of from approximately 60 to approximately 240 standard cubic centimeters per minute ("sccm"). Gas pressure regulators 28 can be adjusted so as to provide a pressure to volumetric pump 34 of from approximately 0.01 to approximately 1.0 psi (0.07 to 6.9 KPa) gauge. Pump 34 may then be operated so as to provide a suitable gas to air flow ratio, such as, for example, approximately 4%.

Gas combustion within calorimeter system 10 takes place within catalytic reactor 50. The gas/air mixture enters catalytic reactor 50 through inlet tube 51. The gas is oxidized within reactor cell 60. The oxidation by-products, together with the excess air, then exit catalytic reactor 50 through exit tube 53.

In order to obtain accurate measurements of the heating value of a gas, it is necessary that reactor cell 60 be substantially isolated from the surrounding environment. Accordingly, reactor cell 60 may be surrounded by an inner shield 54 and/or an outer shield 52.

Inner shield 54 may, for example, comprise a substantially cylindrical stainless steel tube which is approximately 4.4 inches (11.18 cm) long and 1.5 inches (3.81 cm) in diameter. Inner shield 54 is actively maintained at a substantially constant temperature suitably below the operating temperature of reactor cell 60 (such as, for example, approximately 400°C), by means of a heater 56 and a temperature sensor 58.

The temperature difference between inner shield 54 and reactor cell 60 can be adjusted to determine the amount of heat loss to reactor cell 60 and thereby allow control of reactor temperature and gas flow rates for optimum calorimeter operation. For example, decreasing the temperature of inner shield 54 will increase the heat loss to reactor cell 60. Consequently, an increased gas flow rate will be required to maintain reactor cell 60 at the same temperature.

Outer shield 52, on the other hand, may be passive in nature. For example, outer shield 52 may comprise a Division I passive aluminum shield which is configured as a substantially cylindrical structure approximately 9 inches (22.86 cm) long and 5 inches (12.7 cm) in diameter. Such a shield may, for example, remain at a temperature less than approximately 80° C.

In order to be effective, a catalyst must normally be maintained at an elevated temperature. The combustion of gas within reactor cell 60 generally provides sufficient heat to maintain the catalyst at an appropriate elevated temperature. In addition, reactor cell 60 can be provided with a heater 66 to assist in temperature control. When platinum or palladium is being used as a catalyst, reactor cell 60 should preferably be maintained between 400° C and 600° C. Accordingly, heater 66 and/or the heat generated by gas combustion may be used to maintain reactor cell 60 at approximately 450° C to 500° C.

Reactor cell 60 also contains a temperature sensor 68 for sensing the temperature within reactor cell 60. If calorimeter system 10 is being operated in an isoperibol fashion, the temperature measured by sensor 68 can be used directly to indicate the amount of heat liberated by oxidation of the sample gas within reactor cell 60. When operating calorimeter system 10 isothermally, on the other hand, temperature sensor 68 is used to monitor the temperature within reactor cell 60, while the volume of gas provided to reactor cell 60 is carefully measured and controlled in response to sensor 68 so as to maintain reactor cell 60 at a substantially constant temperature. The measured temperature or gas flow information can then be used, as will be described in greater detail below, to determine the NHV of the sample gas.

As mentioned previously, the GHV of a gas is equal to the NHV plus the heat of condensation of the water vapor produced during oxidation of the gas.

Accordingly, in order to determine the GHV of a gas, calorimeter system 10 includes a humidity cell 36 which is connected to the outlet 53 of catalytic reactor 50. Humidity cell 36 measures the relative humidity of the exhaust stream leaving catalytic reactor 50.

Relative humidity measurements, as well as the temperature coefficient of humidity sensors, are highly temperature dependent. It is also desirable to avoid water condensation within the humidity sensor. Accordingly, humidity cell 36 also preferably includes a heater 38 and a temperature sensor 40 so that humidity cell 36 can be maintained at an appropriate operating temperature, thereby substantially avoiding condensation of exhaust water vapor while ensuring stability of the humidity measurement. For example, humidity cell 36 may be maintained at a substantially constant temperature of about 65° C.

After passing through humidity cell 36, the exhaust stream from catalytic reactor 50 enters a water trap 42 which gathers any condensed water vapor. The exhaust stream then leaves calorimeter system 10 through exhaust tube 44.

As mentioned above, it is desirable that catalytic reactor 50 be maintained in a controlled environment. A controlled environment is, for example, essential when using calibration gasses with calorimeter system 10, since changes in environmental temperature can significantly affect the properties of a calibration gas (as in the case of low temperature gas condensation). Accordingly, the enclosure housing calorimeter system 10 may be temperature regulated by means of a heater 47, a fan 46 and a temperature sensor 48. A suitable fan 46 may, for example, comprise a 24 volt DC brushless 4 inch (10.16 cm) diameter fan which is commercially available from a number of different sources.

As further illustrated in Figure 1, calorimeter system 10 of the present invention may advantageously be controlled by means of a computer 100. In such case, computer 100 can be used both to control the various component parts of calorimeter system 10 and to compute the NHV, GHV, and specific gravity of a sample gas. Total energy flow and/or other useful parameters can likewise be calculated by computer 100 if computer 100 is also provided with pipeline volume flow information or other necessary data. A suitable computer 100 may, for example, be based upon either a Motorola 6809 processor or an Intel 80C52 processor.

As depicted in Figure 1, computer system 10 will obtain its power from some external power source 103 (either AC or DC) through a suitable power supply 102. Computer 100 then communicates in conventional fashion with various devices which interface with either a user or with the electrical component parts of calorimeter system 10.

Some of the devices with which computer 100 may communicate are illustrated schematically in Figure 1 as boxes 104 through 118. As shown, computer 100 may be used to activate one or more alarm signals 104 (such as, for example, an LED), upon the occurrence of certain specified conditions. Computer 100 may also control gas valve toggles 105 (such as those commonly designated in the industry as VN10KM devices), which operate gas valves 16. A mass flow interface 106 may also be in communication with computer 100 to convey information obtained from mass flow meter 24.

Computer 100 may also control the temperature within the enclosure housing calorimeter system 10 through a temperature controller 107. Temperature controller 107 would in turn control the operation of fan 46 and heater 47 and would receive temperature data from temperature sensor 48. It will be appreciated that such temperature data can then be conveyed to computer 100 such that an alarm 104 may be activated by computer 100 if the temperature within the enclosure exceeds a specified maximum level (such as, for example, 60° C).

Computer 100 may also direct the operation of a stepping motor driver 108 which controls stepping motor 32 of volumetric pump 34. In this way, computer 100 can be used to control the volume of gas which is introduced to the catalytic reactor 50. Advantageously, the temperature within reactor cell 60 of catalytic reactor 50 can be controlled and monitored by computer 100 through a reactor temperature controller 109. The information obtained by computer 100 from temperature controller 109 can then be used to adjust stepping motor driver 108 so as to maintain a substantially constant combustion temperature within reactor cell 60, if desired.

Computer 100 can also be connected to a temperature controller 110 to control and monitor the temperature of the inner shield 54 of catalytic reactor 50. Similarly, a temperature controller 112 can be used to control and monitor the temperature of humidity cell 36.

A humidity front end interface 111 can be used to provide computer 100 with information about the relative humidity of the exhaust stream from catalytic reactor 50. Other analog inputs may also be provided to computer 100 through a suitable auxiliary analog input interface 113. For example, it may in some cases be desirable to provide computer 100 with volume flow meter data for total energy flow or "therm" calculation by connecting interface 113 to a pipeline volume flow meter.

Computer 100 can, in addition, control various devices which interface with a user. A display 114 (such as, for example, a 16 character by 2 line LCD device), may be used to display the results of calculations to determine the NHV, GHV, and specific gravity of a sample gas. A keypad 116 (such as, for example, a 4 X 4 matrix keypad), may be provided to enable a user to communicate with computer 100. Further, a suitable terminal or modem 118 may be provided to permit operation and control of computer 100 from a remote location.

It will be appreciated by those skilled in the art that a wide variety of different specific component parts may be suitable for use in connection with calorimeter system 10 of the present invention. Inlets 12 and 14, as well as the other gas conduits included within calorimeter system 10, may be any suitable gas conduit. For example, such conduits may comprise 1/8 inch (0.32 cm) stainless steel tubing.

By way of illustration, but not limitation, the table below sets forth some of the specific component parts which are suitable for use in connection with the calorimeter system 10 illustrated in Figure 1. It will be appreciated, however, that numerous alternative component parts could be used without in any way affecting the performance of calorimeter system 10 and without in any way departing from the spirit of the present invention.

## Partial Part List For Calorimeter System

| Component | Reference Numbers | Manufacturer/ Distributor | Part No./ Description |
|---|---|---|---|
| Gas Valves | 16 | Angar | AM-055-1-24 |
| Mass Flow Meter | 24 | Tylan | 380 |
| Pressure Reg. | 28, 30 | Brooks | 2904 B |
| Volumetric Pump | 34 | FMI | "Lab Pump" |
| Humidity Cell and Humidity Front End | 36, 111 | Vaisala | HMP 123B |
| Heaters | 38, 47, 56, 66 | Omega | "Omega Clad" (0.040-0.63 inch or 1 - 16 mm dia) |
| Temperature Sensors | 40, 48, 58, 68 | YSI | 500 ohm Pt RTD |

(a HyCal 1000 ohm Pt RTD would also be suitable for sensors 40, 48 and 58)

### 2. The Catalytic Reactor Cell

The reactor cell 60 of catalytic reactor 50 may have any of a wide variety of different configurations. However, acceptable configurations should be configured so as to provide for substantially complete oxidation of a sample gas. Therefore, careful attention should be paid to the following two design criteria.

First, reactor cell 60 should accommodate a relatively high space velocity. Accordingly, reactor cell 60 should be designed so as to maximize the catalyst surface area which is available to the sample gas. This may be done in a number of ways. For example, temperature sensor 68 within reactor cell 60 may be completely coated with the catalyst. Alternatively, an even greater surface area can be obtained by coating the catalyst onto a plurality of beads having a porous surface structure. Suitable beads may, for example, be formed of alumina. Temperature sensor 68 may then be placed within a bed of such beads. A substantially similar result may be obtained by coating a porous monolithic structure (such as, for example, a ceramic material), with the catalyst and then allowing the sample gas to pass through the monolith.

Suitable catalysts include, for example, platinum group metals (i.e., platinum, palladium, rhodium, iridium, rubidium, and osmium). The presently preferred catalysts from this group are platinum, palladium, and platinum/palladium mixtures. So-called promoters which enhance oxidation at lower temperatures may also be used, such as, for example, copper, cobalt, nickel, and/or cerium, or their oxides.

A catalyst may be coated onto a suitable structure in a number of different ways. For example, platinum and/or palladium catalysts may be provided by soaking a preformed support (such as, for example, numerous spheres or a porous monolith) in concentrated aqueous solutions of the metal chloride. The catalysts are then calcined at approximately 600° C in air before use.

In addition, one should also provide a means within reactor cell 60 for maintaining the temperature at sufficiently high levels that substantially complete oxidation of the sample gas occurs. For example, if platinum is used as a catalyst and if space velocities of about 50 to 100 per second are used, the temperature within reactor cell 60 should be at least 400° C. Under these conditions, more than 99% of the sample gas will be oxidized upon coming into contact with the catalyst. Effective oxidation of the sample gas may also make it desirable to consider preheating the gas before it comes into contact with the catalyst.

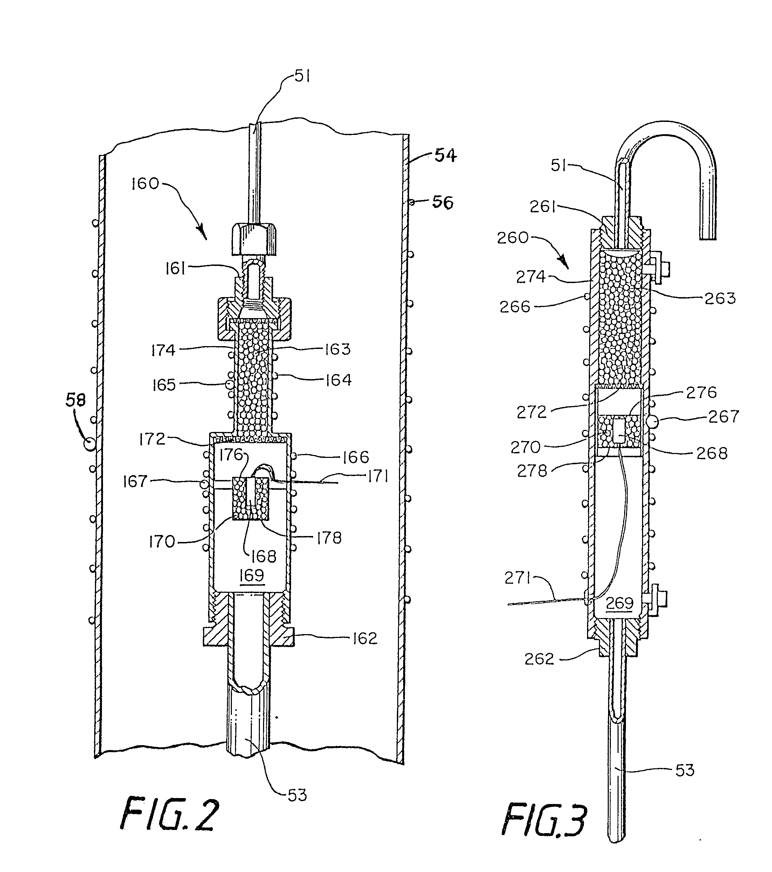

Three of the presently preferred configurations for reactor cell 60 are illustrated in Figures 2 - 7. These configurations are set forth here by way of illustration only. It will be readily appreciated by those skilled in the art that numerous other acceptable configurations are possible.

Referring now to Figure 2, reactor cell 160 is shown within shield 54. Heater 56 comprises a wire which is wound around shield 54 in a spiral fashion, and a temperature sensor 58 is provided on the surface of shield 54 to monitor the shield temperature.

Reactor cell 160 comprises a housing 74 which is connected by means of suitable couplings 161 and 162 to inlet tube 51 and outlet tube 53, respectively. As illustrated in Figure 2, housing 174 may comprise a unitary structure which has two substantially cylindrical sections with different diameters. Housing 174 could, of course, also be a unitary cylindrical structure with a single diameter.

Within housing 174 adjacent coupling 161 a plurality of preheating beads 163 are provided. Beads 163 are maintained within the upper portion of housing 174 by means of a screen 172 or some other suitable structure. A heater 164 and a temperature sensor 165 are wound around housing 174 so as to surround beads 163.

Immediately below screen 172 is an open space 169. A plurality of beads 170 are provided within space 169 and may be retained by means of screens 176 and 178. Beads 170 are coated with a catalyst such as for example platinum, palladium, or a mixture of platinum and palladium. Beads 170 thus form a catalyst bed through which the gas within space 169 passes so as to promote combustion of the sample gas within space 169.

A sensor 168 is provided within catalyst bed 170 so as to sense the temperature within catalyst bed 170. The electrical leads from sensor 168 exit housing 174 as shown at 171 and are thereafter connected to appropriate electrical circuitry. The portion of housing 174 which surrounds catalyst bed 170 is also provided with a heater 166 and a temperature sensor 167.

In operation, a mixture of gas and air enters reactor cell 160 through conduit 51. The gas first passes through preheater beads 63 which raise the temperature of the gas and air mixture to a suitable temperature such as, for example, 400° C. The gas then passes through screen 172 into space 169 where it comes into contact with catalyst bed 170. Space 169 is maintained at an appropriate temperature such as, for example, 400° C so as to promote combustion of the sample gas within space 169 as it comes into contact with catalyst bed 170. The combustion by-products, together with excess air, then exit reactor cell 160 through exit conduit 53.

A second presently preferred embodiment 260 of a reactor cell for use in connection with the present invention is illustrated in Figure 3. It will be appreciated that reactor cell 260 will also be enclosed by an inner shield 54 as illustrated in Figures 1 and 2. However, for brevity and simplicity, inner shield 54 is not depicted in Figure 3.

Reactor cell 260 is connected to inlet tube 51 and outlet tube 53 by means of suitable connectors 261 and 262, respectively. Reactor cell 260 comprises a substantially cylindrical housing 274 and is substantially completely surrounded by a spirally wound heater wire 266. A temperature sensor 267 is also provided on the surface of housing 274 so as to be substantially adjacent to temperature sensor 268 within housing 274, as shown.

The top portion of housing 274 serves as a preheater section. This section of housing 274 is filled with a plurality of preheater beads 263 and are retained by a means of a suitable screen 272. Below screen 272, a catalyst bed 270 is provided. Catalyst bed 270 comprises a plurality of beads which are coated with a suitable catalyst, such as, for example, platinum and/or palladium. A temperature sensor 268 is provided within bed 270, and the electrical lead from sensor 268 exits housing 274 at 271 as shown. Also, the beads which comprise bed 270 are retained by means of suitable screens 276 and 278.

The operation of reactor cell 260 is substantially the same as the operation of reactor cell 160 described above in connection with Figure 2. Gas enters reactor cell 260 through conduit 51. The gas and air mixture then passes through preheater beads 263 through screen 272 and through catalyst bed 270. As the gas passes through catalyst bed 270 the gas is oxidized, and the oxidation by-products and excess air then exit reactor cell 260 through conduit 53.

As with reactor cell 160, it is desirable to maintain reactor cell 260 at a sufficiently high temperature to ensure substantially complete oxidation of a sample gas as it passes through reactor cell 260. Thus, for example, heater 266 and sensor 267 may be operated so as to maintain housing 274 at approximately 400° C.

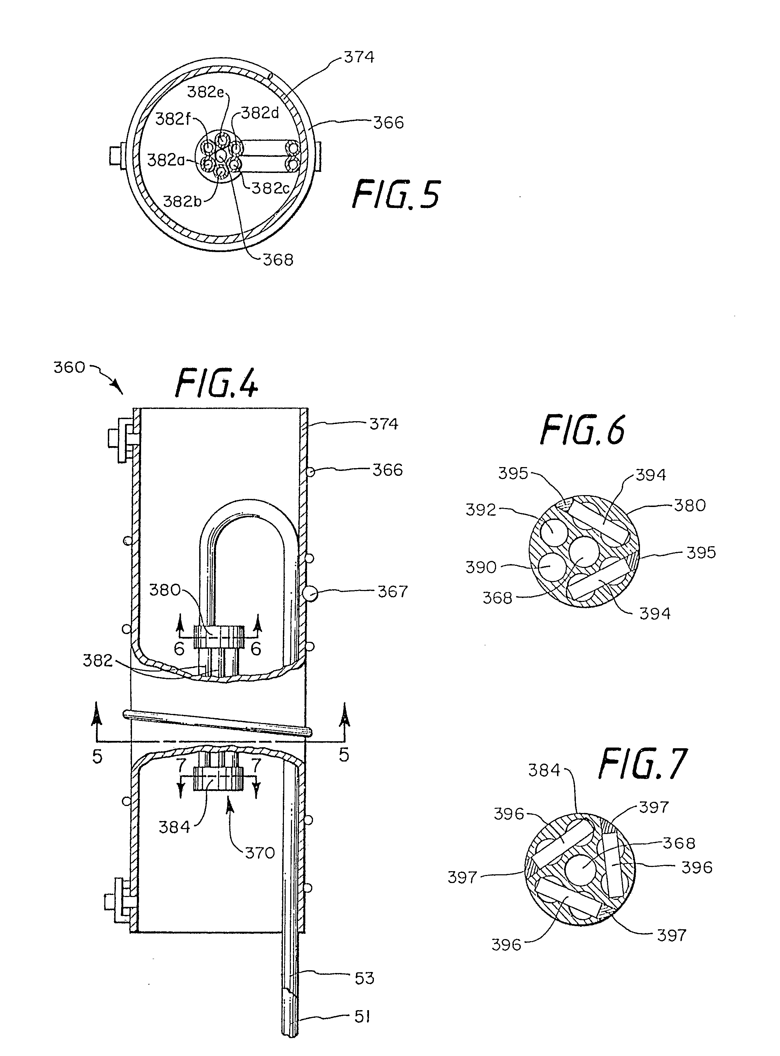

A third presently preferred embodiment 360 of a reactor cell within the scope of the present invention is illustrated in Figures 4 - 7. In reactor cell 360, the catalytic bed 170 is provided within a plurality of tubes 382, and the gas flow is directed sequentially through each tube so as to provide for prolonged contact between the sample gas and the catalyst.

As illustrated in Figure 4, reactor cell 360 comprises a housing 374 which is substantially cylindrical. A heating wire 366 is wound spirally around housing 374, and a temperature sensor 367 is provided adjacent housing 374. Inlet 251 and outlet 253 are each connected to catalyst bed 370 contained within tubes 382 by means of manifolds 380 and 384.

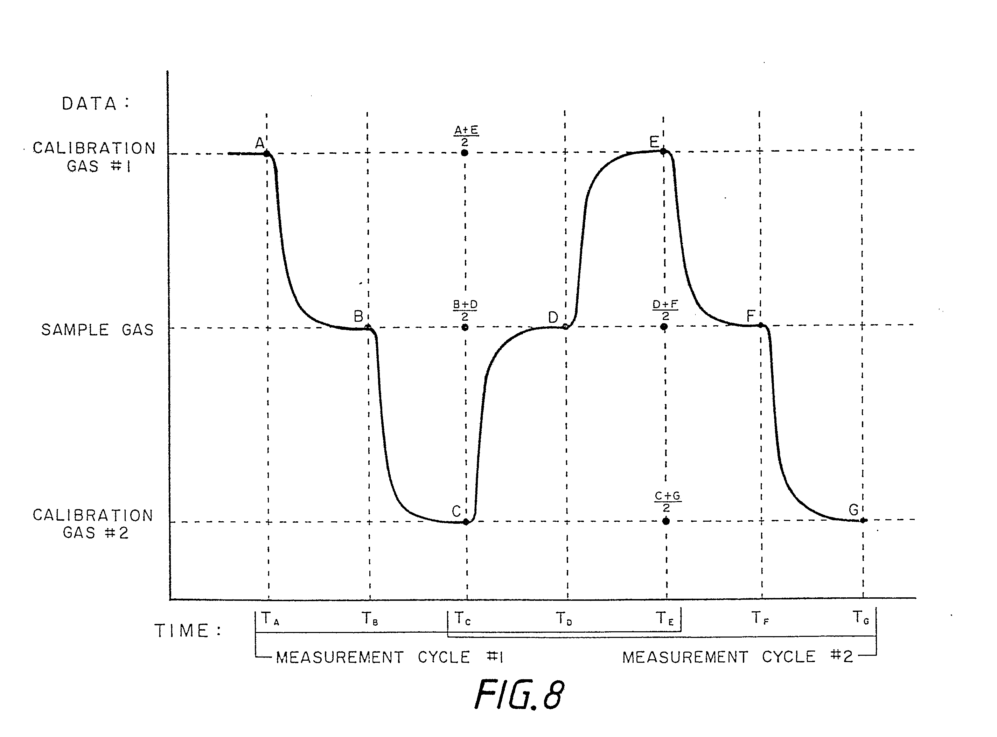

The way in which the gas and air mixture travel through catalyst bed 370 can be seen with reference to Figures 5, 6 and 7. The gas and air mixture first passes through manifold 380 illustrated in Figure 6 through opening 390. As shown in Figure 5, the gas and air mixture then travel downwardly through tube 382a. Upon reaching bottom manifold 384 illustrated in Figure 7, the gas and air mixture travel through a passageway 396 (the outer opening of which has been sealed by a plug 397, as shown), to the adjacent tube 382b in a clockwise direction. The gas and air mixture then travel again upwardly toward top manifold 380, through a passageway 394 (which is sealed by a plug 395, as shown), and into tube 382c. The mixture continues to travel between top manifold 380 and bottom manifold 384 until it reaches opening 392 of top manifold 380 through tube 382f. The gas and air mixture then passes through opening 392 to outlet conduit 53 and out of reactor cell 360.

As shown in Figures 5, 6, and 7, a temperature sensor 368 is positioned in the center of tubes 382. Tubes 382 are filled with catalyst coated beads. Thus, as the gas and air mixture travel through the several tubes 382, the gas is oxidized as it comes into contact with the catalyst, and sensor 368 senses the heat generated as a result of such oxidation.

The various component parts of the reactor cells described in connection with Figures 2 - 7 above can be made of any suitable material. For example, the preheating beads 163 and 263 may be formed of any inert, moderately heat conductive material. The beads in the catalyst beds 170, 270, and 370 (within tubes 382) may be formed of a conventional catalyst substrate material having a porous surface, such as, for example, alumina. The various screens which retain the beads may be formed of stainless steel as may the housing 174, 274, and 374 of the various reactor cells. Tubes 382 illustrated in Figures 4 - 7, together with manifolds 380 and 384, may likewise be formed of stainless steel.

### 3. Calorimeter System Operation

The calorimeter system 10 of the present invention may be operated so as to provide reliable measurements by using an appropriate calibration gas. This may be done using conventional linear interpolation methods. It is presently preferred that at least two calibration gases are used which have known heating values at the upper and lower limits of the normal operating range of calorimeter system 10.

For example, referring to Figure 1, a first calibration gas may be admitted through valve 16c into catalytic of reactor 50 for a given period of time, such as, for example, 10 minutes. Various measurements can then be made upon the first calibration gas. For example, a reading may be obtained from mass flow meter 24, a temperature reading may be obtained from temperature sensor 68, a volume reading may be obtained by means of stepping motor driver 108 and volumetric pump 34, and a relative humidity measurement may be obtained by humidity cell 36.

After a suitable time interval, valve 16c can be closed, and a sample gas can be admitted through valve 16a. Similar measurements are then obtained with respect to the sample gas for a like interval of time.

A second calibration gas may then be admitted through valve 16d with similar measurements again being made. Thereafter, the sample gas could again be admitted through valve 16a for a second set of measurements. Finally, the first calibration gas could once again be admitted through valve 16c for a second set of measurements.

Figure 8 illustrates a measurement cycle such as that described above. Importantly, the NHV, GHV, and specific gravity of the two calibration gases are known. Thus, using interpolation techniques, the NHV, GHV, and specific gravity of the sample gas may be determined.

Figure 8 illustrates a typical data plot in which the burn sequence was calibration gas #1 (A), sample gas (B), calibration gas #2 (C), sample gas (D), calibration gas #1 (E), sample gas (F), calibration gas #2 (G), and so forth. The ordinate in Figure 8 is plotted in whichever units the data is collected, be it reactor temperature, pump speed, or relative humidity. The abscissa is plotted in arbitrary units of time (Ta, Tb, Tc, etc.), which correspond to the data points A, B, C, etc. Figure 8 shows 7 data points which comprise two measurement cycles, each consisting of 5 consecutive data points (i.e., measurement cycle #1 consists of points A, B, C, D and E, while measurement cycle #2 consists of points C, D, E, F, and G).

Data is compared only at the center of each measurement cycle so that the time element of instrumentation drift is minimized over the cycle. For example, point Tc is the center of cycle #1; calibration gas #1 data points A and E are averaged at Tc to form $\frac{A+E}{2}$ ; sample as data points B and D are averaged at Tc to form $\frac{B+D}{2}$ ; data point C remains unmodified, of course. Similarly, measurement cycle #2 has its center at point Te, so data point E is unmodified but data points D and F are averaged at Te to form $\frac{D+F}{2}$ , and data points C and G are averaged at Te to form $\frac{C+G}{2}$ . This technique provides a continuous 2 point calibration which calibrates the zero offset and gain of the instrumentation as well as its drift with time.

The distance $\frac{A+E}{2}$ - C calibrates the sensitivity of the instrument, since the data points A and E are generated by calibration gas #1 and data point C is generated by calibration gas #2. Distance $\frac{B+D}{2}$ - E represents the unknown sample gas signal which is compared to distance $\frac{A+E}{2}$ - C to obtain the ratio of sample gas signal to calibration gas signal. This ratio is then compared to the known properties of the calibration gases to obtain the corresponding property of the sample gas, referred to data point C.

For example, to obtain sample gas NHV using data from measurement cycle #1, the following equation is used:

$$NHV(sample) = \frac{\dfrac{B + D}{2} - C}{\dfrac{A + E}{2} - C} (NHV\#1 - NHV\#2) + NHV\#2 \qquad (1)$$

where

NHV(sample) = NHV of sample gas

A = reactor measurement during first time interval

B = reactor measurement during second time interval

C = reactor measurement during third time interval

D = reactor measurement during fourth time interval

E = reactor measurement during fifth time interval

NHV#1 = NHV of first calibration gas

NHV#2 = NHV of second calibration gas

A similar equation could, of course, be developed for measurement cycle #2.

The specific application of equation (1) will depend upon the manner in which calorimeter system 10 is being operated. For isoperibol operation, pump 34 of calorimeter system 10 would be operated so as to provide a substantially fixed volume of gas to catalytic reactor 50 per given unit of time. The reactor measurements (A, B, C, C, E) during the various time intervals would in such case be a measure of the temperature within reactor cell 60 as measured by temperature sensor 68.

Alternatively, pump 34 and/or heater 66 could be selectively controlled so as to maintain the temperature within reactor cell 60 substantially constant. For such isothermal operation of the calorimeter system 10, the various reactor measurements (A, B, C, D, E) would be a measurement of the speed of pump 34 as controlled by stepping motor driver 108. Alternatively, the various reactor measurements could be the sample loop cycling rate (if a sampling loop is used in place of pump 34) and/or the reactor control heater power level required to maintain a constant temperature within reactor cell 60.

Gross heating value calculations are based on the ratio of gross heating value (GHV) to net heating value (NHV). This ratio depends entirely on the latent heat of vaporization of water produced by combustion of the sample of gas, which latent heat is directly proportional to the number of water molecules in (or relative humidity of) the exhaust stream of the calorimeter. This assumes the use of a uniform amount of dry combustion air. The use of one or two calibration gasses with known GHV to NHV ratios can null out instrumentation inaccuracies and drifts, providing relative accuracies to $\pm$ 0.3 BTU/SCF.

For example, by measuring the relative humidity (RH) and using the net heating value (NHV) calculated previously, the ratio of gross to net heating value can be determined, using the following equation for isothermal operation of calorimeter system 10 during measurement cycle #1:

$$GHV(sample) = NHV(sample) \left| \frac{\dfrac{RHB + RHD}{2} - RHC}{\dfrac{RHA + RHE}{2} - RHC} \right|$$

$$x \quad \left( \frac{GHV\#1}{NHV\#1} - \frac{GHV\#2}{NHV\#2} \right) + \frac{GHV\#2}{NHV\#2} \qquad (2)$$

where

GHV(sample) = GHV of sample gas

RHA = relative humidity measurement during first time interval

(RHB through RHE are similar measurements during subsequent time intervals)

GHV#1 = GHV of first calibration gas

GHV#2 = GHV of second calibration gas

Specific gravity measurement and calculation requires mass flow and volumetric flow measurements for the sample and calibration gasses. By dividing mass flow by volumetric flow, a mass per unit volume number may be obtained for each gas. Then, by using the known specific gravity numbers of the calibration gasses as references, a linear interpolation similar to that applied in the NHV calculation may be employed for measurement cycle #1, as follows:

$$\text{SP GR (sample)} = \frac{\dfrac{B\ SP\ GR + D\ SP\ GR}{2} - C\ SP\ GR}{\dfrac{A\ SP\ GR + E\ SP\ GR}{2} - C\ SP\ GR}$$

$$\times\ (SP\ GR\ \#1 - SP\ GR\ \#2) + SP\ GR\ \#2 \quad (3)$$

where
SP GR(sample) = specific gravity of sample gas
A SP GR = specific gravity measurement during first time interval = mass flow rate divided by pump volume speed
(B SP GR through E SP GR are similar measurements during subsequent time intervals) SP GR #1 = specific gravity of first calibration gas
SP GR #2 = specific gravity of second calibration gas

Equation (3) set forth above for calculation of specific gravity uses information derived from mass flow meter 24 and volumetric pump 34. Mass flow meter 24 will provide information relating to the mass of gas entering calorimeter system 10 per unit time. Pump 34 can be used to obtain information about the volume of gas entering calorimeter system 10 per unit time. This data can be combined in the manner set forth above to determine the specific gravity of the sample gas.

Various other types of operating modes are, of course, possible, as will be appreciated by those skilled in the art. Other operating conditions and modes of operation may require slight modifications to the equations set forth above, such as, for example, the use of pressure and/or temperature correction coefficients or the infrequent use of only one calibration gas. However, for the specific isoperibol and/or isothermal modes described, the foregoing equations will provide accurate readings of NHV, GHV, and specific gravity for various sample gases.

4. Summary

From the above discussion, it will be appreciated that the present invention provides a sensitive gas calorimeter which is both small and inexpensive. The gas calorimeter of the present invention need occupy only about one cubic foot, and it can be manufactured at only a fraction of the cost of conventional calorimeters. In addition, the gas calorimeter of the present invention is capable of continuously monitoring the NHV, GHV, and specific gravity of a sample gas and is easy to operate, calibrate and maintain. It is believed that these features make the present invention much more suitable for field installation than conventional prior art calorimeter devices.

The calorimeter system of the present invention also operates on the basis of catalytic combustion, and thus no high temperature flame is required to oxidize the sample gas. As noted above, the calorimeter system of the present invention can operate on relatively low gas flow rates, as compared with typical prior art calorimeters, and the gas to air volume ratio can be below the lower inflammability limit of most sample gasses. It is, therefore, believed that the present invention is much more safe and economical than the prior art calorimeter devices now in use.

The invention may be embodied in other specific forms without departing from its spirit or essential characteristics. The described embodiments are to be considered in all respects only as illustrative and not restrictive. The scope of the invention is, therefore, indicated by the appended claims, rather than by the foregoing description. All changes which come within the meaning and range of equivalency of the claims are to be embraced within their scope.

**Claims**

1. A gas calorimeter system for measuring the heating values of a sample gas, comprising:
a reactor cell for oxidizing the sample gas;
a porous substrate positioned within the reactor cell;
a catalyst coating the porous substrate for oxidizing the sample gas;
means for metering a desired volume of the sample gas into the reactor cell;
means for metering a desired volume of air into the reactor cell; and
means for sensing the temperature within the reactor cell.
2. A gas calorimeter system as claimed in claim 1 wherein the catalyst comprises a platinum group

11

metal.

3. A gas calorimeter system as claimed in claim 1 or claim 2 wherein the reactor cell comprises a promoter for oxidizing the sample gas.

4. A gas calorimeter system as claimed in any one of the preceding claims wherein the porous substrate is configured as a plurality of beads.

5. A gas calorimeter system as claimed in claim 4 wherein the reactor temperature sensing means is positioned so as to be substantially surrounded by said beads.

6. A gas calorimeter system as claimed in claim 4 further comprising a plurality of conduits which are interconnected such that the gas and air entering the reactor cell passes sequentially through each conduit, and wherein the porous beads are located within the conduits and the reactor temperature sensing means is positioned so as to be in thermal contact with the conduits.

7. A gas calorimeter system as claimed in claim 6 wherein the conduits are oriented substantially parallel to one another in a substantially cylindrical configuration and wherein the reactor temperature sensing means is positioned so as to be substantially surrounded by the conduits.

8. A gas calorimeter system as claimed in any one of the preceding claims further comprising heater means for maintaining the temperature within at least a portion of the reactor cell adjacent the catalyst within the range of from approximately 400°C to approximately 600°C.

9. A gas calorimeter system as claimed in any one of the preceding claims further comprising a temperature shield which substantially surrounds the reactor cell.

10. A gas calorimeter system as claimed in claim 9 wherein the temperature shield comprises a metal enclosure.

11. A gas calorimeter system as claimed in claim 10 further comprising means for heating the metal enclosure and means for sensing the temperature of the metal enclosure.

12. A gas calorimeter system as claimed in any one of the preceding claims wherein the gas metering means and the air metering means are configured so as to provide a gas-to-air flow ratio within the range of from approximately one percent to approximately five percent.

13. A gas calorimeter system as claimed in any one of the preceding claims further comprising means for measuring the flow of combustion mass into the reactor cell.

14. A gas calorimeter system as claimed in any one of the preceding claims further comprising means for measuring the amount of water produced by oxidation of the sample gas.

15. A gas calorimeter system as defined in claim 14 wherein the water measuring means comprises a humidity cell for measuring the relative humidity of gaseous matter exiting the reactor cell.

16. A gas calorimeter system as claimed in claim 15 wherein the water measuring means further comprises means for selectively controlling the temperature within the humidity cell so as to minimize condensation of water within the humidity cell and maintain stability of the relative humidity measurement.

17. A gas calorimeter system as claimed in any one of the preceding claims further comprising:
a supply of calibration gas having a known net heating value and gross heating value; and
means for selectively metering a desired volume of the calibration gas into the reactor cell.

18. A gas calorimeter system as claimed in any one of the preceding claims further comprising means for preheating the gas and air entering the reactor cell.

19. A method for measuring the net heating value and the gross heating value of a sample gas, comprising the steps of:
metering a known volume of the sample gas into a reactor cell:
oxidizing the sample gas within the reactor cell in the presence of an excess of air;
monitoring the temperature within the reactor cell; and
measuring the amount of water produced by oxidation of the sample gas.

20. A method as claimed in claim 19 wherein the gas oxidizing step comprises directing the sample gas into the vicinity of a catalyst.

21. A method as claimed in claim 19 or claim 20 wherein the water measuring step comprises measuring the relative humidity of gaseous matter exiting the reactor cell.

22. A method as claimed in any one of claims 19 to 21 wherein the volume of the sample gas metered into the reactor cell in the gas metering step remains substantially constant and wherein the net heating value of the sample gas is determined by measuring the temperature within the reactor cell resulting from oxidation of the sample gas.

23. A method as claimed in any one of claims 19 to 21 wherein the volume of the sample gas metered into the reactor cell in the gas metering step is selectively controlled in response to the reactor cell temperature monitoring step such that the temperature within the reactor cell is maintained at a substantially constant level, and wherein the volume of the sample gas which is metered into the reactor cell is measured to determine the net heating value of the sample gas.

24. A method as claimed in any one of claims 19 to 23, further comprising the steps of:
metering a known volume of a calibration gas into the reactor cell, the calibration gas having a known net heating value and gross heating value;
oxidizing the calibration gas within the reactor cell in the presence of an excess of air; and
wherein the net heating value and the gross heating value of the sample gas are determined by comparing measurements made during oxidation of the sample gas to measurements made during oxidation of the calibration gas.

25. A method as claimed in any one of claims 19 to 24 further comprising the step of measuring the flow of combustion mass into the reactor cell, whereby the specific gravity of the sample gas can be determined.

EP 0 304 266 A2

FIG.1

51

54

160

56

161

174 — 163

165 — 164

58

172 — 166

176

167 — 171

170 — 178

168

169

162

53

*FIG.2*

51

261

260

274

266 — 263

272 — 276

270 — 267

278 — 268

271

269

262

53

*FIG.3*

FIG.5

FIG.4

FIG.6

FIG.7

FIG.8